# EUROPÄISCHE PATENTSCHRIFT

(11) **EP 2 504 012 B1**
(45) Veröffentlichungstag und Bekanntmachung des Hinweises auf die Patenterteilung: **23.03.2016**
(21) Anmeldenummer: 10784299.9
(22) Anmeldetag: 24.11.2010
(51) Int. Cl.: A61K 31/56, A61K 47/44, A61K 9/06, A61K 9/00

(54) **VERWENDUNG EINES TRITERPENHALTIGEN OLEOGELS ZUR WUNDHEILUNG**
USE OF AN OLEO GEL CONTAINING TRITERPENE FOR HEALING WOUNDS
UTILISATION D'UN OLÉOGEL CONTENANT DU TRITERPÈNE POUR LA CICATRISATION DE PLAIES

(30) Priorität: 24.11.2009 DE 102009047092
(43) Veröffentlichungstag der Anmeldung: 03.10.2012
(73) Patentinhaber: Birken AG, 75223 Niefern-Öschelbronn (DE)
(72) Erfinder: SCHEFFLER, Armin, 75229 Niefern-Öschelbronn (DE)
(74) Vertreter: Westphal, Mussgnug & Partner Patentanwälte mbB
(86) Internationale Anmeldenummer: PCT/EP2010/068157
(87) Internationale Veröffentlichungsnummer: WO 2011/064271

(56) Entgegenhaltungen:
- EP-A1- 1 872 788
- WO-A1-01/72315
- WO-A1-2005/123037
- WO-A2-2009/090394
- US-A- 6 022 890
- MAQUART F-X ET AL: "STIMULATION OF COLLAGEN SYNTHESIS IN FIBROBLAST CULTURES BY A TRITERPENE EXTRACTED FROM CENTELLA ASIATICA", CONNECTIVE TISSUE RESEARCH : AN INTERNAT. JOURNAL, NEW YORK, NY : INFORMA HEALTHCARE, US, Bd. 24, 1. Januar 1990 (1990-01-01), Seiten 107-120, XP000946035, ISSN: 0300-8207, DOI: 10.3109/03008209009152427
- SHRIKHANDE B K ET AL: "DEVELOPMENT AND EVALUATION OF ANTI-INFLAMMATORY OLEOGELS OF BOSEWELLIA SERRATA (GUGUL) AND CURCUMA LONGA (TURMERIC)", INDIAN DRUGS, INDIAN DRUG MANUFACTURERS' ASSOCIATION, IN, Bd. 38, Nr. 12, 1. Dezember 2001 (2001-12-01), Seiten 613-616, XP008005371, ISSN: 0019-462X

## Beschreibung

Die vorliegende Erfindung betrifft die Verwendung eines triterpenhaltigen Oleogels.

Gele sind feindisperse Systeme aus einer flüssigen und einer festen Phase, wobei die feste Phase ein zusammenhängendes dreidimensionales Gerüst bildet und die beiden Phasen sich vollständig durchdringen. Man unterscheidet im wesentlichen zwischen hydrophilen Gelen und hydrophoben Gelen. Letztere werden auch als Oleogele bezeichnet. Oleogele basieren auf einer unpolaren Flüssigkeit, beispielsweise einem Öl, einem Wachs oder einem Paraffin, der ein Gelbildner zur Erzielung der gewünschten physikalischen Eigenschaften beigefügt ist.

Derartige Oleogele können je nach Zusammensetzung unterschiedlichsten Zwecken dienen.

Besonders im pharmazeutischen Bereich finden Oleogele Verwendung für topische Anwendungen. Bei diesen pharmazeutischen Oleogelen ist ein Gelbildner neben den pharmazeutisch wirksamen Substanzen in dem Gel vorhanden. Ein häufig verwendeter Gelbildner für pharmazeutische Oleogele ist hochdisperses Siliziumdioxid, das unter dem Handelsnamen Aerosil^{®} erhältlich ist. Oleogele besitzen eine ausgeprägte Thixotropie, d.h. sie verflüssigen sich bei mechanischer Einwirkung und verfestigen sich anschließend wieder. Andere Gele, beispielsweise Gele mit Pektin als Gelbildner, vernetzen unter Säureeinwirkung, wieder andere gelieren temperaturabhängig, wie beispielsweise Gelatine.

In der DE 10 2004 030 044 A1 ist die Verwendung eines hochdispersen Triterpens als Oleogelbildner und ein Oleogel mit einem hochdispersen Triterpen als Oleogelbildner beschrieben.

Bekannte Substanzen zur Wundheilung, d.h. zur Heilung von Hautwunden beim Menschen und bei Säugetieren, sind beispielsweise Dexpanthenol oder Kamillenauszüge. Um diese Substanzen zu anwendbaren Arzneimitteln zu verarbeiten, sind allerdings Hilfsstoffe, wie Emulgatoren, Löse- oder Konservierungsmittel, erforderlich. Diese Hilfsstoffe können jedoch wundheilungsstörend wirken und können außerdem bei manchen Patienten zu allergischen Reaktionen führen.

Die WO 2005/123037 A1 beschreibt ein triterpenhaltiges Oleogel, das ein triterpenhaltiges Pulver als Oleogelbildner aufweist. Dieses Oleogel eignet sich zur Behandlung von Hautkrankheiten, wie beispielsweise aktinische Keratosen.

Marquart F-X et al. "Stimulation of Collagen Synthesis in Fibroblast Cultures by a Triterpene Extracted From Cantella Asiatica", Connective Tissue Research: An Internat. Journal, New York, NY; Informa Healthcare, US, Bd. 24, 1. Januar 1990(1990-01-01), Seiten 107-120, ISSN: 0300-8207, beschreibt die Verwendung eines aus Cantella Asiatica gewonnenen Extrakts zur Wundheilungsstimulation.

WO 2009/090394 A2 beschreibt die Verwendung eines aus Capsicum gewonnenen Extrakts für die Verwendung bei der Behandlung verschiedener Hautkrankheiten wie beispielsweise Exzemen oder Epdermolysis bullosa.

Aufgabe der vorliegenden Erfindung ist es, ein wirksames und einfach herzustellendes Präparat zur Wundheilung der Haut, insbesondere zur Heilung chronischer Wunden, zur Verfügung zu stellen, das darüber hinaus unter Allergiegesichtspunkten gut verträglich ist.

Diese Aufgabe wird durch eine Verwendung nach Anspruch 1 gelöst. Ausgestaltungen und Weiterbildungen sind Gegenstand von Unteransprüchen.

Erfindungsgemäß wird zur Wundheilung der Haut ein Oleogel eingesetzt, das eine unpolare Flüssigkeit und wenigstens ein triterpenhaltiges Pulver als Oleogelbildner enthält bzw. ein solches Oleogel wird zur Herstellung eines Arzneimittels zur Wundheilung eingesetzt.

Ein solches Oleogel eignet sich grundsätzlich zur Heilung aller Arten von Wunden, wie beispielsweise zur Heilung von Wunden, die durch äußere Einflüsse, wie Unfälle, hervorgerufen wurden, und auch zur Heilung von Wunden, insbesondere von chronischen Wunden, die durch Hautkrankheiten bedingt sind. Eine solche Hautkrankheit ist beispielsweise die genetisch bedingte Hautkrankheit Epidermolysis bullosa (EB). Bei Menschen, die an dieser Krankheit erkrankt sind, ist die mechanische Verbindung zwischen den unterschiedlichen Hautschichten unzureichend ausgebildet, so dass bereits durch geringfügige mechanische Belastungen Blasen und Wunden entstehen können.

Triterpene, wie z.B. Betulin, Lupeol, Betulinsäure, Oleanolsäure und ähnliche Verbindungen, sind nachwachsende Rohstoffe, die in vergleichsweise hoher Konzentration in Birkenrinde vorkommen, die jedoch auch in anderen Pflanzen oder Pflanzenbestandteilen, wie z.B. in Rosmarinblättern, Misteln oder Apfelschalen, vorkommen. Betulin, Betulinsäure, Lupeol und Oleanolsäure sind pentazyklische Triterpene, von denen die drei zuerst genannten ein Lupan-Gerüst aufweisen und von denen die Oleanolsäure ein Oleanengerüst aufweist. Das charakteristische Merkmal der Lupan-Gruppe ist ein Ring mit fünf Kohlenstoffatomen innerhalb des pentazyklischen Systems, der eine α-Isopentenylgruppe an der Position C-19 besitzt.

Als Oleogelbildner in dem Oleogel eignet sich ein beliebiges Triterpen bzw. eine beliebige Triterpenzusammensetzung, das in Pulverform vorliegt und das ausreichend fein pulverisiert ist, um als Oleogelbildner zu wirken. Eine Triterpenzusammensetzung umfasst zwei oder mehr unterschiedliche Triterpene. Gemäß einem Beispiel beträgt die mittlere Partikelgröße des wenigstens einen Triterpens in dem Oleogelbildner vorzugsweise zwischen 20nm und 50µm, besonders bevorzugt weniger als 10µm.

Vorteilhafterweise liegt außerdem eine homogene Partikelgrößenverteilung vor, worunter im nachfolgenden zu verstehen ist, dass ein Anteil an Sekundäragglomeraten in dem hochdispersen triterpenhaltigen Pulver weniger als 20 Gew.% beträgt.

Der in Form eines mikronisierten triterpenhaltigen Pulvers vorliegende Oleogelbildner kann neben Triterpenen, wie beispielsweise Betulin, Betulinsäure, Lupeol oder Allobetulin, auch einen Anteil anderer Stoffe umfassen, beispielsweise solche Stoffe, die in triterpenhaltigen Pflanzenbestandteilen, wie beispielsweise Birkenrinde, aus denen Triterpene extrahiert werden können, ebenfalls in einem gewissen Anteil natürlich vorhanden sind. Der Triterpenanteil in dem erfindungsgemäßen Oleogelbildner beträgt vorzugsweise mehr als 80 Gew.%, besonders bevorzugt mehr als 90 Gew.% bezogen auf das Gewicht des Oleogelbildners. Vorteilhafterweise beträgt der Betulinanteil bezogen auf den Triterpenanteil dabei mehr als 60 Gew.%, und insbesondere mehr als 80 Gew.%.

Das als Oleogelbildner verwendete wenigstens eine Triterpen kann mittels herkömmlicher nicht-kontinuierlicher Extraktionsverfahren (Batch-Verfahren) oder mittels herkömmlicher kontinuierlicher Verfahren aus Pflanzen oder Pflanzenbestandteilen, wie z.B. aus Birkenrinde, Rosmarin, Misteln oder Apfelschalen, extrahiert werden, so dass auf weitere Ausführungen hierzu verzichtet werden kann. Kontinuierliche Verfahren zur Gewinnung von Triterpenen aus Pflanzenbestandteilen, insbesondere von Betulin aus Birkenrinde, sind beispielsweise in der WO 2001/72315 A1 oder der WO 2004/016336 A1 beschrieben.

Sofern das wenigstens ein Triterpen enthaltende Pulver nach der Extraktion nicht mit der für die Gelbildungseigenschaften erforderlichen Dispergierbarkeit, mittleren Partikelgröße und homogenen Partikelgrößenverteilung vorliegt, kann das Pulver verschiedenen Verfahren unterzogen werden, um zu der gewünschten Partikelgröße, Homogenität und Dispergierbarkeit zu gelangen. Hierfür sind verschiedene Verfahren bekannt: Wenn die Partikelgröße in dem Pulver zu hoch ist, eignen sich Prall- oder Gravitationsverfahren zur Verkleinerung der Partikel. Darüber hinaus besteht die Möglichkeit, das Pulver in einem geeignetem Lösungsmittel, beispielsweise Tetrahydrofuran (THF), zu lösen und anschließend erneut zu kristallisieren. Diese Kristallisation kann beispielsweise durch Sprühtrocknung oder Abkühlen eines gesättigten Lösungsmittels erfolgen. Die Partikelgröße kann dabei über die Kristallisationsbedingungen eingestellt werden. Die Kristallisationsbedingungen sind bei einer Sprühtrocknung beispielsweise vom Durchmesser einer Düse, über welche das Triterpen-Lösungsmittel-Gemisch versprüht wird, und von der Temperatur und dem Druck in einer Kammer, in die das Gemisch gesprüht wird, abhängig. Beim Kristallisieren durch Abkühlen einer gesättigten Lösung sind die Kristallisationsbedingungen vom zeitlichen Temperaturgradienten während des Abkühlens und von der Triterpenkonzentration in der Lösung abhängig. Schließlich besteht auch die Möglichkeit ein vorhandenes Pulver zu klassieren, um ein Pulver mit einer gewünschten Größenverteilung zu erhalten.

Der Anteil der unpolaren Flüssigkeit in dem Oleogel beträgt vorzugsweise zwischen 88 Gew.% und 94 Gew.%, und der Anteil des triterpenhaltigen Pulvers beträgt vorzugsweise zwischen 6 Gew.% und 12 Gew.%.

Als unpolare Flüssigkeit für das Oleogel eignen sich beliebige unpolare Flüssigkeiten, wie beispielsweise pflanzliche, tierische oder synthetische Öle, Wachse und Paraffine. Die unpolare Flüssigkeit ist beispielsweise ein pflanzliches Öl, das aus einem der folgenden ausgewählt ist: Sonnenblumenöl, Olivenöl, Avocadoöl, Mandelöl.

Der Vorteil dieser halbfesten Zubereitung in Form eines Oleogels liegt in der Einfachheit seiner Rezeptur, wobei das Triterpen gleichzeitig als pharmazeutisch wirksame wundheilende Substanz und als Gelbildner funktioniert, so dass auf zusätzliche Gelbildner verzichtet werden kann. Das Oleogel ist dadurch besonders als Wundheilmittel für allergiegefährdete Haut geeignet.

Selbstverständlich besteht jedoch auch die Möglichkeit, dem Oleogel neben dem im Gelbildner vorhandenen Triterpen weitere pharmazeutisch aktive Substanzen in pharmakologisch wirksamer Konzentration beizufügen, wie Dexpanthenol oder Kamillenauszüge. Das Oleogel mit der unpolaren Flüssigkeit und dem triterpenhaltigen Pulver als Oleogelbildner stellt eine ideale Grundlage für solche Stoffe dar, da es lipophile Substanzen aufnehmen kann. So besteht auch die Möglichkeit, der unpolaren Flüssigkeit bereits eine lipophile pharmazeutisch aktive Substanz beizufügen noch bevor das Oleogel durch Zugabe des triterpenhaltigen Pulvers hergestellt wird.

Außerdem kann ein wässriger Auszug mit Hilfe des Oleogels zu einer stabilen Emulsion verarbeitet werden.

Die vorliegende Erfindung wird nachfolgend anhand von Beispielen, insbesondere unter Bezugnahme auf die beigefügten Figuren erläutert.
- Figur 1: veranschaulicht die Partikelgrößenverteilung eines Beispiels eines hochdispersen Oleogelbildners in einem zur Wundheilung geeigneten Oleogel.
- Figur 2: veranschaulicht den Wundheilungsfortschritt bei der Wundheilung nach dem "Porcines ex-vivo Wundmodell" unter Einfluss eines triterpenhaltigen Oleogels.

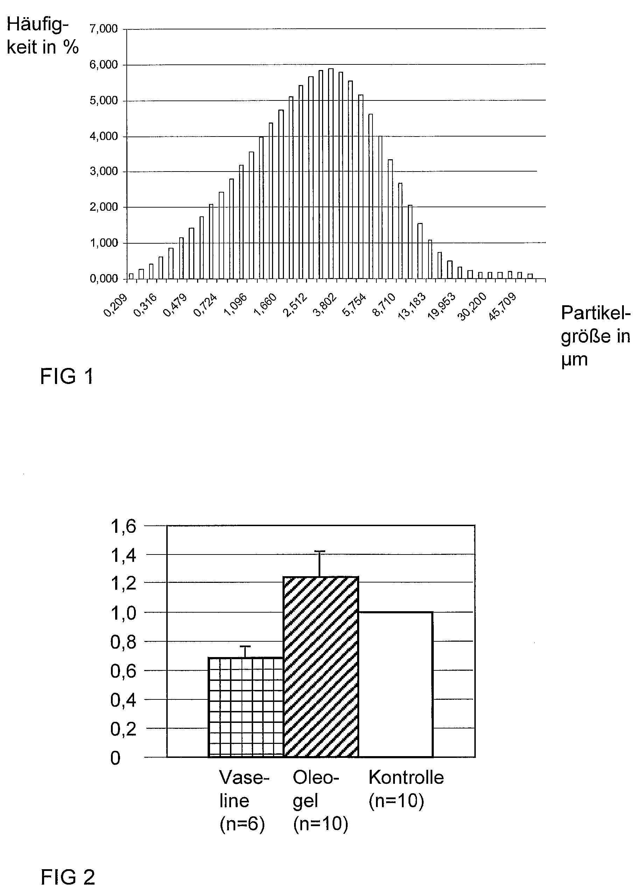

Figur 1 veranschaulicht die Partikelgrößenverteilung eines Beispiels eines hochdispersen Oleogelbildners in einem zur Wundheilung geeigneten Oleogel. In dem Beispiel liegt die Partikelgröße zwischen 0,5µm und 40µm, das Maximum der Größenverteilung liegt zwischen 8µm und 10µm. Gemäß gaschromatographischer Analyse enthält dieses Pulver 85 Gew.% Betulin, 5 Gew.% Betulinsäure, 3% Oleanolsäure, 0,7 Gew.% Lupeol und 6,3 Gew.% übrige Triterpenderivate.

Unter Verwendung dieses Pulvers als Gelbildner wurde ein Oleogel hergestellt, indem das Pulver mit 10 Gew.% bezogen auf das Gesamtgewicht des Oleogels mit Sonnenblumenöl vermischt wurde. Ergebnis war ein stabiles halbfestes Gel mit stark ausgeprägter Thixotropie (Oleogel-S10). Dieses Gel wird im nachfolgend als Oleogel-S10 bezeichnet, wobei "S10" auf einen Anteil von 10% des triterpenhaltigen Pulvers in dem Oleogel hinweist. Zusätzlich wundheilende Substanzen sind in dem Oleogel-S10 nicht vorhanden.

Ein Oleogel mit einem hochdispersen triterpenhaltigen Pulver als Oleogelbildner eignet sich als Wundheilmittel zur Behandlung beliebiger Hautwunden des menschlichen Körpers. Solche Wunden können Wunden sein, die durch Unfälle hervorgerufen wurden, wie z.B. Schnitt- oder Schürfwunden oder auch Brandwunden. Solche Wunden können jedoch auch Wunden sein, die gezielt zu therapeutischen Zwecken erforderlich sind, wie z.B. Wunden nach einer Spalthautentnahme oder Wunden nach einer Laserbehandlung, wie z.B. einer Laserbehandlung zur Entfernung von Tätowierungen oder von Hautwucherungen. Ein Oleogel mit einem hochdispersen triterpenhaltigen Pulver als Oleogelbildner eignet sich auch als Wundheilmittel zur Behandlung von Wunden, die durch Hautkrankheiten, wie z.B. Epidermolysis bullosa, bedingt sind.

### 1. Beispiel:

Die Wundheilwirkung eines triterpenhaltigen Oleogel (OleogelS10) wurde anhand des "Porcines ex-vivo Wundmodells" wie es Gegenstand der DE 103 17 400 B4 ist, getestet. Von Hautproben des Schweineohrs mit einem Durchmesser von 6mm wurde hierbei in einem kleineren kreisförmigen Bereich die Epidermis und der obere Teil der Dermis entfernt. Bei einer ersten Gruppe von 10 Proben wurde in die hierdurch entstandenen Wunden einmalig für 48 Stunden 10 µl Oleogel eingebracht, bei einer zweiten Gruppe von 6 Proben wurde 10 µl Vaseline, die als Vergleichspräparat diente, appliziert und eine dritte Gruppe von 10 Proben blieb als Kontrollgruppe unbehandelt. Nach 48 Stunden wurden die Hautproben fixiert und anschließend histologisch untersucht.

Die Untersuchung zeigte an Hand des Reepithelialisierungsfortschritts eine verbesserten Wundheilung bei den mit dem Oleogel behandelten Proben im Vergleich zu den anderen Proben. Der Wundheilungsfortschritt ist in Figur 2 in Form eines Balkendiagramms grafisch dargestellt. Dabei zeigt der linke Balken den Wundheilungsfortschritt für Vaseline, der rechte Balken den der Kontrollprobe, und der mittlere Balken den für das Oleogel. Wie ersichtlich ist, führt die Behandlung mit dem Oleogel zu einem rascheren Wundheilungsfortschritt im Vergleich sowohl zu einer Nicht-Behandlung, als auch zu einer Behandlung mit Vaseline.

Das Oleogel zeigte außerdem eine ähnlich gute Erhaltung der Morphologie des Wundrands wie bei den nicht behandelten Proben. Die Zahl der proliferativen Zellen in der regenerierenden Epidermis war unter dem Einfluss des Oleogels im Vergleich zur unbehandelten Probe tendenziell erniedrigt, am Wundrand jedoch gleich, während hier die Vaseline statistisch signifikant weniger proliferative Zellen aufwies.

### 2. Beispiel:

Patientin (w), 3 Jahre
Diagnose: Epidermolysis bullosa junctionalis non-Herlitz
- Wundstatus vor Therapie:
   - flache fibrinös belegte chronische Wunde, Thorax rechts
   - Keine Heilungstendenz über mehr als vier Wochen
   - Größe: 13,63 cm²
- Therapie bisher:
   - täglicher Verbandwechsel, Wundversorgung mit Mepitel und Mepilex transfer; regelmäßige Oktenisept-Hautdesinfektion.
- Nebendiagnosen:
   - MRSA Kolonisation in 6 Lokalisationen, kein Abstrich aus der behandelten
   - Eisenmangelanämien
   - Alimentäre Dystrophie
   - chronische Schmerzen
- Therapiebeginn 15.07.:
   - tägliche Oleogel-S10 Applikation, Mepilex transfer Wundverband
   - Begleittherapie: antiseptische Bäder
   - Medikation: Ibuprofen 3-mal 80 mg, Tavigil (2x 5ml)
- Wundbefund am 17.07.:
   - Größe: 9,58 cm² (ca. 30% Wundflächenverkleinerung)
   - flache nicht belegte Wunde,
   - Epithelisation an den Wundrändern und Ausbildung einer Epithelbrücke

### 3. Beispiel:

Patient (m), 4 Jahre
Diagnose: Epidermolysis bullosa simplex
- Ausgangsbefund:
   - annulär sich ausbreitende Bläschen und Krusten
   - Rücken und beide Flanken
   - bestehend seit 5 Wochen
   - massiver Juckreiz
- Therapie bisher:
   - Bepanthen, Fucidine und Mepilex ohne anhaltende Verbesserung
- Therapiebeginn am 12.12.2008:
   - Behandlung mit Octisept Lösung
   - 2x tägl. Oleogel-S10; Abdeckung der Wunde mit Mepilex transfer.;
   - Medikation: Fenistil Tropfen; Aerius Sirup; Excipial U Lipolotio 2 x tgl.; 5% Thesit in Unguentum leniens bei Bedarf tagsüber.
- Befund am 18.12.2008:
   - weniger Bläschen und Krusten
      - Juckreiz gelindert
- Nachbeobachtung 02.05.2009 unter Fortführung der Oleogelbehandlung:
   - Abheilung und Ausbleiben des Juckreizes

### 4. Beispiel:

Patientin (w), 12 Jahre
Diagnose: Rezessiv dystrophe Epidermolysis bullosa
- Wundstatus vor Therapie:
   - Innenknöchel links: flache, leicht entzündete, schmerzende (Visuelle Analog Skala 0 - 100: VAS 50), exsudierende (VAS 50) Wunde; seit 10.04.09 bestehend
   - Knie rechts ventral: flache, leicht entzündete, leicht schmerzende (VAS 15), exsudierende (VAS 40) Wunde; seit 13.04.09 bestehend
- Therapie bisher:
   - Urgotül; Mepilex Lite
- Therapie ab 16.04.2009:
   - beide Wunden: Urgotül; Mepilex Lite, täglicher Verbandwechsel
   - Innenknöchel links zusätzlich mit Oleogel-S10
- Wundbefund am 22.05.2009:
   - Beide Wunden abgeheilt
   - Knöchel links (Oleogel-S10): epithelisiert (VAS 100); leicht gerötet (VAS 8), nicht schmerzend oder juckend (VAS 0)
   - Knie rechts (Kontrolle): epithelisiert mit Restkruste (VAS 90); leicht gerötet (VAS 8), leicht schmerzend (VAS 10) und juckend (VAS 5)

### 5. Beispiel:

Patient (m), 57 Jahre
Diagnose: Epidermolysis bullosa dystrophica inversa
- Wundstatus vor Therapie am 18.11.2008:
   - flache, fibrinös belegte Wunden
   - Größe: 9,48cm²
   - skrotal rechts und links,
   - keine Abheilung über mehr als 3 Monate
- Therapie bisher:
   - verschiedenste Salben und Cremes, keine Verbesserung
- Nebenbefunde:
   - Wundbesiedlung mit Staph. aureus, Proteus
   - Diabetes mellitus, insulinpflichtig
- Therapiebeginn 18.11.2008:
   - Oleogel-S10: 2x täglich
   - Wundauflage Mepilex transfer
- Wundstatus am 24.11.2008:
   - fast vollständig abgeheilte, flache, fibrinös belegte Wunde
   - Größe: 0,65cm²
- Nachbeobachtung:
   - Verschlechterung nach Absetzen von Oleogel-S10
   - Therapieversuch mit Mirfulan Creme, kein wesentliche Besserung
   - Therapieversuch mit Imlan Creme Pur, nur geringe Besserung
   - Nach erneuter Therapie mit Oleogel-S10, Abheilung

Die Wundbehandlung mit einem Oleogel, das ein triterpenhaltiges Pulver als Oleogelbildner umfasst, bewirkt bereits nach wenigen Tagen einen einsetzbaren Heilungsprozess und eine Verkleinerung der Wunden, und somit eine deutliche Linderung. Bei anhaltender Behandlung bewirkt das Oleogel ein vollständiges Abheilen der Wunde, und zwar insbesondere auch das Abheilen chronischer Wunden, bei denen zuvor spontan kein Heilungsprozess eingesetzt hat. Das Oleogel fördert bei der Wundheilung insbesondere die Reepithelialisierung, kann also insbesondere bei der Wundheilung während der Reepithelialisierungsphase eingesetzt werden.

Außer den in den Beispielen genannten äußeren Epithelien eignet sich das Oleogel auch zur Wundheilung an innerer Epithelien (Schleimhäute), wie z.B. im Nasen-, Magen oder Genitalbereich. Das Oleogel kann unbedenklich oral verabreicht werden.

Die im Zusammenhang mit Figur 1 erläuterte Triterpenzusammensetzung (Zusammensetzung I) steht lediglich beispielhaft für eine Triterpenzusammensetzung, die als Bestandteil bzw. als Oleogelbildner eines Oleogels eine wundheilende Wirkung besitzt. Die wundheilende Wirkung eines triterpenhaltigen Oleogels ist dabei selbstverständlich nicht auf ein Oleogel mit einer solchen speziellen Triterpenzusammensetzung beschränkt. Beispielhaft sind nachfolgend drei weitere Triterpenzusammensetzungen angegeben, unter Verwendung derer Oleogele hergestellt wurden, deren wundheilende Wirkung anhand des "Porcines ex-vivo Wundmodell" verifiziert wurde. Nachfolgend sind für diese Zusammensetzungen, die als Zusammensetzungen II-IV bezeichnet sind, deren Hauptbestandteile und deren jeweiliger Anteil in Gew.% angegeben.

### Zusammensetzung II:

| | |
|---|---|
| Betulin: | 86,85 Gew.% |
| Lupeol: | 3,94 Gew.% |
| Betulinsäure: | 3,52 Gew.% |
| Erythrodiol: | 0,77 Gew.% |
| Oleanolsäure: | 0,62 Gew.% |

### Zusammensetzung III:

| | |
|---|---|
| Betulin: | 78,32 Gew.% |
| Lupeol: | 7,18 Gew.% |
| Betulinsäure: | 3,46 Gew.% |
| Erythrodiol: | 0,77 Gew.% |
| Oleanolsäure: | 0,63 Gew.% |

### Zusammensetzung IV:

| | |
|---|---|
| Betulin: | 60,50 Gew.% |
| Lupeol: | 25,43 Gew.% |
| Betulinsäure: | 1,68 Gew.% |
| Erythrodiol: | 1,47 Gew.% |
| Oleanolsäure: | 0,48 Gew.% |

Wie insbesondere das Beispiel der Zusammensetzung III zeigt, die einen vergleichsweise niedrigen Betulinanteil besitzt, muss für eine gute Wundheilung nicht notwendigerweise ein hoher Betulinanteil vorhanden sein.

Der gemeinsame Anteil an Betulin und Lupeol in den Zusammensetzungen I-IV liegt jeweils über 80 Gew.%, insbesondere über 85 Gew.%. In welchem Anteil die einzelnen Triterpene vorhanden sind, ist insbesondere davon abhängig aus welchen Pflanzen oder Pflanzenteilen das triterpenhaltige Pulver gewonnen wurde. Eine gute Wundheilung ist allerdings nicht von der speziellen Zusammensetzung des triterpenhaltigen Pulvers abhängig. Vielmehr scheinen Oleogele mit beliebigen Triterpenen als Oleogelbildner, gute Wundheilungseigenschaften zu besitzen.

Außer Sonnenblumenöl sind selbstverständlich beliebige weitere Fette oder Öle zur Herstellung des Oleogels geeignet, die für den Mensch oder für Säugetiere nicht toxisch sind bzw. die medizinisch anwendbar sind.

## Patentansprüche

1. Oleogel zur Verwendung bei der Wundheilung, das eine unpolare Flüssigkeit und ein triterpenhaltiges Pulver als Oleogelbildner enthält,
wobei das triterpenhaltige Pulver Betulin und Lupeol aufweist und ein gemeinsamer Anteil an Betulin und Lupeol in dem triterpenhaltigen Pulver größer als 80 Gew.% ist.

2. Oleogel zur Verwendung nach Anspruch 1 zur Verwendung bei der Wundheilung in der Reepithelialisierungsphase.

3. Oleogel zur Verwendung nach Anspruch 1 oder 2, das aufweist:
eine unpolare Flüssigkeit mit einem Anteil zwischen 80 Gew.% und 99 Gew.% bezogen auf das Gesamtgewicht des Gels,
ein triterpenhaltiges Pulver als Oleogelbildner mit einem Anteil zwischen 1 Gew.% und 20 Gew.% bezogen auf das Gesamtgewicht des Gels.

4. Oleogel zur Verwendung nach Anspruch 3, bei dem der Anteil der unpolaren Flüssigkeit zwischen 88 Gew.% und 94 Gew.% und der Anteil des Oleogelbildners zwischen 6 Gew.% und 12 Gew.% beträgt.

5. Oleogel zur Verwendung nach einem der vorangehenden Ansprüche, bei dem die unpolare Flüssigkeit ein pflanzliches, tierisches, mineralisches oder synthetisches Öl ist.

6. Oleogel zur Verwendung nach Anspruch 5, bei dem das Öl ein Pflanzenöl ist, das aus einem der folgenden ausgewählt ist: Sonnenblumenöl, Olivenöl, Avocadoöl, Mandelöl.

7. Oleogel zur Verwendung nach einem der vorangehenden Ansprüche bei dem das triterpenhaltige Pulver einen Triterpenanteil von mehr als 80 Gew.% aufweist.

8. Oleogel zur Verwendung nach einem der vorangehenden Ansprüche bei dem das triterpenhaltige Pulver einen Triterpenanteil von mehr als 90 Gew.% aufweist.

9. Oleogel zur Verwendung nach Anspruch 8, bei dem ein Anteil von Betulin an dem Triterpenanteil mehr als 50 Gew.% oder mehr als 60 Gew.% beträgt.

10. Oleogel zur Verwendung nach einem der vorangehenden Ansprüche, bei dem eine mittlere Partikelgröße des triterpenhaltigen Pulvers zwischen 20nm und 50µ beträgt.

11. Oleogel zur Verwendung nach Anspruch 10, bei dem eine mittlere Partikelgröße des triterpenhaltigen Pulvers weniger als 10µ beträgt.

12. Oleogel zur Verwendung nach einem der vorangehenden Ansprüche, zur Verwendung bei der Wundheilung von Wunden bei Epidermolysis bullosa sind.

13. Oleogel zur Verwendung nach einem der Ansprüche 1 bis 11, zur Verwendung bei der Wundheilung von Wunden, die durch Entnahme von Spalthaut entstanden sind.

14. Oleogel zur Verwendung nach einem der Ansprüche 1 bis 11, zur Verwendung bei der Wundheilung von Wunden, die durch eine Laserbehandlung der Haut entstanden sind.

15. Oleogel zur Verwendung nach einem der vorangehenden Ansprüche, das zusätzlich eine wundheilende Substanz umfasst.

16. Oleogel zur Verwendung nach einem der vorangehenden Ansprüche, bei dem der gemeinsame Anteil an Betulin und Lupeol in dem triterpenhaltigen Pulver größer als 85 Gew.% ist.

17. Oleogel zur Verwendung nach einem der vorangehenden Ansprüche, bei dem das triterpenhaltige Pulver weiterhin aufweist: Betulinsäure, Oleanolsäure, Erythrodiol.

## Claims

1. An oleogel for use in wound healing, containing a nonpolar liquid and a triterpene-containing powder as an oleogel forming agent,
wherein the triterpene-containing powder contains betulin and lupeol and a combined fraction of betulin and lupeol in the triterpene-containing powder is greater than 80 wt.-%.

2. The oleogel for use according to claim 1 for use in wound healing in the reepithelialization phase.

3. The oleogel for use according to claim 1 or 2, containing:
a nonpolar liquid with a fraction of between 80 wt.-% and 99 wt.-% based on the total weight of the gel,
a triterpene-containing powder as an oleogel forming agent with a fraction of between 1 wt.-% and 20 wt.-% based on the total weight of the gel.

4. The oleogel for use according to claim 3, in which the fraction of the nonpolar liquid is between 88 wt.-% and 94 wt.-% and the traction of the oleogel forming agent is between 6 wt.-% and 12 wt.-%.

5. The oleogel for use according to any one of the preceding claims, in which the nonpolar liquid is a vegetable, animal, mineral or synthetic oil.

6. The oleogel for use according to claim 5, in which the oil is a vegetable oil selected from one of the following: sunflower oil, olive oil, avocado oil, almond oil.

7. The oleogel for use according to any one of the preceding claims, in which the triterpene-containing powder has a triterpene fraction of more than 80 wt.-%.

8. The oleogel for use according to any one of the preceding claims, in which the triterpene-containing powder has a triterpene fraction of more than 90 wt.-%.

9. The oleogel for use according to claim 8, in which a fraction of betulin in the triterpene fraction is more than 50 wt.-% or more than 60 wt.-%.

10. The oleogel for use according to any one of the preceding claims, in which a mean particle size of the triterpene-containing powder is between 20 nm and 50 µm.

11. The oleogel for use according to claim 10, in which a mean particle size of the triterpene-containing powder amounts to less than 10 µm.

12. The oleogel for use according to any one of the preceding claims, for use in the wound healing of wounds in epidermolysis bullosa.

13. The oleogel for use according to any one of claims 1 to 11, for use in the wound healing of wounds produced by the removal of split skin.

14. The oleogel for use according to any one of claims 1 to 11, for use in the wound healing of wounds caused by laser treatment of the skin.

15. The oleogel for use according to any one of the preceding claims, which also comprises a wound-healing substance.

16. The oleogel for use according to any one of the preceding claims, in which the combined fraction of betulin and lupeol in the triterpene-containing powder is greater than 85 wt.-%.

17. The oleogel 2 for use according to any one of the preceding claims, in which
the triterpene-containing powder also contains: betulic acid, oleanolic acid, erythrodiol.

## Revendications

1. Oléogel destiné à être utilisé dans le cadre de la cicatrisation de plaies, qui contient un liquide non polaire et, en tant qu'agent oléogélifiant, une poudre contenant du triterpène,
sachant que la poudre contenant du triterpène présente de la bétuline et du lupéol et qu'une fraction en bétuline et en lupéol dans la poudre contenant du triterpène est supérieure à 80 % en poids.

2. Oléogel destiné à être utilisé selon la revendication 1, destiné à être utilisé dans le cadre de la cicatrisation de plaies en phase de réépithélialisation.

3. Olégel destiné à être utilisé selon la revendication 1 ou 2, lequel présente :
un liquide non polaire présentant une fraction comprise en 80 % en poids et 99 % en poids, par rapport au poids total du gel
une poudre contenant du triterpène en tant qu'agent oléogélifiant présentant une fraction comprise entre 1 % en poids et 20 % en poids par rapport au poids total du gel.

4. Oléogel destiné à être utilisé selon la revendication 3, dans le cadre duquel la fraction du liquide non polaire présente une valeur comprise entre 88 % en poids et 94 % en poids, et la fraction de l'agent oléogélifiant présente une valeur comprise entre 6 % en poids et 12 % en poids.

5. Oléogel destiné à être utilisé selon l'une quelconque des revendications précédentes, dans le cadre duquel le liquide non polaire est une huile végétale, animale, minérale ou synthétique.

6. Oléogel destiné à être utilisé selon la revendication 5, dans le cadre duquel l'huile est une huile végétale, qui est choisie parmi les huiles suivantes : huile de tournesol, huile d'olive, huile d'avocat, huile d'amande.

7. Oléogel destiné à être utilisé selon l'une quelconque des revendications précédentes, dans le cadre duquel la poudre contenant du triterpène présente une fraction de triterpène supérieure à 80 % en poids.

8. Oléogel destiné à être utilisé selon l'une quelconque des revendications précédentes, dans le cadre duquel la poudre contenant du triterpène présente une fraction de triterpène supérieure à 90 % en poids.

9. Oléogel destiné à être utilisé selon la revendication 8, dans le cadre duquel une fraction de bétuline sur la fraction de triterpène présente une valeur supérieure à 50 % en poids ou supérieure à 60 % en poids.

10. Oléogel destiné à être utilisé selon l'une quelconque des revendications précédentes, dans le cadre duquel une taille moyenne des particules de la poudre contenant du triterpène présente une valeur comprise entre 20 nm et 50 µm.

11. Oléogel destiné à être utilisé selon la revendication 10, dans le cadre duquel une taille moyenne des particules de la poudre contenant du triterpène présente une valeur inférieure à 10 µm.

12. Oléogel destiné à être utilisé selon l'une quelconque des revendications précédentes, destiné à être utilisé dans le cadre de la cicatrisation de plaies en présence d'une épidermolyse bulleuse.

13. Oléogel destiné à être utilisé selon l'une quelconque des revendications 1 à 11, destiné à être utilisé dans le cadre de la cicatrisation de plaies apparues du fait du prélèvement de fascias.

14. Oléogel destiné à être utilisé selon l'une quelconque des revendications 1 à 11, destiné à être utilisé dans le cadre de la cicatrisation de plaies apparues du fait d'un traitement au laser de la peau.

15. Oléogel destiné à être utilisé selon l'une quelconque des revendications précédentes, qui comprend en supplément une substance cicatrisante.

16. Oléogel destiné à être utilisé selon l'une quelconque des revendications précédentes, dans le cadre duquel la fraction commune de bétuline et de lupéol dans la poudre contenant du triterpène est supérieure à 85 % en poids.

17. Oléogel destiné à être utilisé selon l'une quelconque des revendications précédentes, dans le cadre duquel la poudre contenant du triterpène présente un outre : de l'acide bétulinique, de l'acide oléanolique, de l'érythrodiol.
